# EUROPEAN PATENT APPLICATION

(11) **EP 3 566 760 A1**
(43) Date of publication of application: **13.11.2019**
(21) Application number: 18171018.7
(22) Date of filing: 07.05.2018
(51) Int. Cl.: B01D 9/00, B01D 9/02, C07D 207/27

(54) **METHOD FOR NUCLEATING CRYSTALS FROM A SOLUTION IN A CAPILLARY TUBE**

(71) Applicant: Université Libre de Bruxelles, 1050 Bruxelles (BE)
(72) Inventor: SCHEID, Benoit, 1050 BRUXELLES (BE); RIMEZ, Bart, 1500 HALLE (BE); NORRANT-LECOMTE, Edith, 1050 BRUXELLES (BE)
(74) Representative: Pronovem

(57) **Abstract**

Method for nucleating crystals from a solution comprising the steps of:

• injecting in a first capillary (1) tube an under-saturated solution comprising a solvent and a soluble compound to be crystallised;
• changing the local conditions of the solution downstream of the capillary tube (1) to super-saturated conditions above the meta-stable conditions, the transition time of the fluid flowing in the capillary tube between the under-saturated conditions and the super-saturated conditions above the meta-stable conditions being less than 1000 ms.

The present method is preferably used for crystallising Brivaracetam.

## Description

### Field of the invention

The present invention is related to a method for improving nucleation step in a crystallisation process.

### State of the art

Many products in the pharmaceutical and high value chemical industries go through multiple crystallization steps during their development and manufacture. Crystallisation is commonly used in purification of chemicals and controls size, shape and polymorphism of the obtained chemicals.

In the pharmaceutical industry, precisely controlling the size, polymorphism and shape of the particles is also of key importance, as it has an influence on pharmacokinetics and mixing behaviour of active molecules. Usually, size of particles in powders is obtained by a grinding step, also referred to as micronization, which can be problematic, as heating may be introduced in the process by friction effects.

Nowadays, continuous flow synthesis of organic molecules is becoming an area of upmost interest for the pharmaceutical industry and more generally in engineering and chemistry. Continuous phase synthesis and crystallization of high value Active Pharmaceutical Ingredients (API) have been successfully reported for molecules such as artemisinin, imatinib, efavirenz and others (for example Cogoni G.; de Souza B.P.; Frawley P.J., Chem. Eng. Sci. 2015, 138, 592-599). Several continuous crystallization methods have been described and developed commercially, like e.g. mixed suspension, mixed product removal setups (MSMPR, see Alvarez A.J. Singh A.; Myerson A.S., Cryst. Growth Des. 2011,11, 4392-4400.), and impinging jet crystallizers (see Metzger L. ; Kind M., Chem. Eng. Sci., 2015, 133, 91-105).

Many organic molecules exhibit polymorphism and pseudo-polymorphism, where a compound can adopt more than one crystal form without or with solvent molecules incorporated in the crystal lattice. Selective crystallization of metastable crystals becomes then mandatory to retain the requested end-product polymorph. The approach for selective crystallization is based on seeding locations or spontaneous nucleation of the targeted form in these domains and controlling the cooling or antisolvent addition rate, or both, so that the combined effects only promote the nucleation of the desired (meta)stable crystalline form. Many approaches are described in literature using single and mixed solvents, supercritical crystallization, seeding strategies, capillary crystallization, polymer- and substrate-induced heteronucleation, and others.

Therefore, there is a need to develop a continuous and seedless process to crystallise chemicals from solution wherein the obtained crystalline structure is well defined, and wherein the size and shape of the particles is controlled, so that a grinding (or micronization) step can be avoided.

### Summary of the invention

The present invention discloses a method for nucleating crystals from a solution comprising the steps of:
- Injecting in a first capillary tube an under saturated solution comprising a solvent and a soluble compound to be crystallised;
- changing the local conditions of the solution downstream of the capillary tube to supersaturated conditions above the metastable conditions, the transition time of the fluid flowing in the capillary tube between the under saturated conditions and the supersaturated conditions above the metastable conditions being less than 1000 ms, preferably below 100 ms, even more preferably less than 10 ms.

Preferred embodiments of the present invention disclose at least one, or an appropriate combination of the following features:
- the transition to supersaturated conditions is obtained by cooling down the capillary wall to temperature below the metastable temperature, and/or injecting a antisolvent in the injected stream, and/or injecting a cooled solvent, the cooled or antisolvent liquid being injected by a second capillary tube having essentially the same dimension as the first capillary;
- the first capillary tube (1) has an internal diameter comprised between 1 mm and 100µm, preferably between 800µm and 500µm;
- the method comprises the additional step of initially setting the supersaturation between -0.1 (i.e. under saturated) and the high concentration limit of the metastable conditions, supersaturation being defined as the relative difference between the local concentration of the compound to be crystallized and the solubility of the compound to be crystallized;
- the capillary tube comprises static mixing zone(s) improving thermal, antisolvent diffusion and shear rate;
- the static mixing zone comprises section restrictions, preferably, from one to four restrictions;
- the inner diameters of the restrictions are comprised between 100 and 900µm preferably between 250 and 750µm, and a length comprised between 1mm and 50cm;
- the static mixing zone is located upstream of the transition zone between under saturated or metastable conditions and supersaturated conditions above the metastable conditions;
- the supersaturation above the metastable conditions is comprised between 1.6 and 30, preferably between 2 and 18;
- the capillary length is comprised between 15cm and 10m;
- the flow in the capillary length is continuous;
- the flow rate of the under saturated solution is comprised between 2 and 200 mL/min, preferably between 10 and 50mL/min;
- the method is performed as a continuous open loop process;
- the soluble compound is an active pharmaceutical ingredients preferably selected from the group consisting of acetylsalicylic acid, paracetamol, etiracetam, piracetam, brivaracetam, lamivudine, ketamine, lactic acid, lactate esters, antiretroviral drugs (like abacavir, atazanavir, darunavir, didanosine, efavirenz, emtricitabine, tenofovir, ...), ibuprofen, artesunate, entecavir, zanamivir, artemether, and their mixture;
- the solvent is selected from the group consisting of water, alcohol, acid, ether, ketone, alcane, amide, ester and their mixtures;
- the solvent is selected from the group consisting of water, methanol, ethanol, propanol, isopropanol, hexanol, ethyl acetate, isopropyl acetate, methyl isoutyl ketone, methyl isopropyl ketone, acetic acid, acetone, acetonitrile, dimethyl formamide, dimethyl sulfoxide, pentane, hexane, cyclohexane, tetrahydrofuran, benzene, toluene, diethyl ether, and their mixtures.

The invention is also related to a method for crystallising a compound from an under saturated solution comprising the steps of nucleating crystals of the compound by the method according to any of the previous claims and growing the obtained nuclei in a metastable supersaturated solution.

Another aspect of the invention is related to a method of seeding a supersaturated solution by injecting nuclei obtained by the method for nucleating crystals from a supersaturated solution according to the invention.

### Short description of the drawings

Fig. 1 represents an example of crystallisation setup according to the invention.
Fig. 2 shows the results of a simulation of the temperature gradient in a capillary tube of an example of the invention.

### List of reference symbols

1: molecule/solvent solution entry line, 1 mm inner diameter or less;
2: Optional antisolvent entry line, 1 mm or less inner diameter;
3: restriction setup, various inner diameters placed in series;
4: cooling bath;
5: optional vessel with stirrer;
6: coolant;
7: collecting fluid;
8: output.

### Detailed description of the invention

The device and method of the invention is related to the controlled crystallisation of crystallisable soluble organic molecules such as active pharmaceutical ingredients. The device and method of the invention is particularly suitable for molecules where the crystallisation is mainly limited by the nucleation process. Preferred molecules are pharmaceutical ingredients and more general small organic molecules selected from the group consisting of acetylsalicylic acid, paracetamol, etiracetam, piracetam, brivaracetam, lamivudine, ketamine, lactic acid, lactate esters, antiretroviral drugs (like abacavir, atazanavir, darunavir, didanosine, efavirenz, emtricitabine, tenofovir, ...), ibuprofen, artesunate, entecavir, zanamivir and artemether, and others.

The device and method of the invention is used to continuously nucleate and grow crystals of organic molecules (solute) in solution inside capillaries with inner diameter equal or less than 1 millimeter for industrial production. The very small size of the capillary ensures that saturation conditions can be varied very fast, getting in less than 1 second from under saturated conditions or metastable conditions (i.e. supersaturated conditions, but without spontaneous nucleation) to strongly supersaturated solutions, above the spontaneous nucleation threshold. For instance, surface to volume ratio in such system permits very fast and efficient cooling on one side, and the very small volume inside the capillary permits high concentration gradient improving antisolvent mixing on the other side.

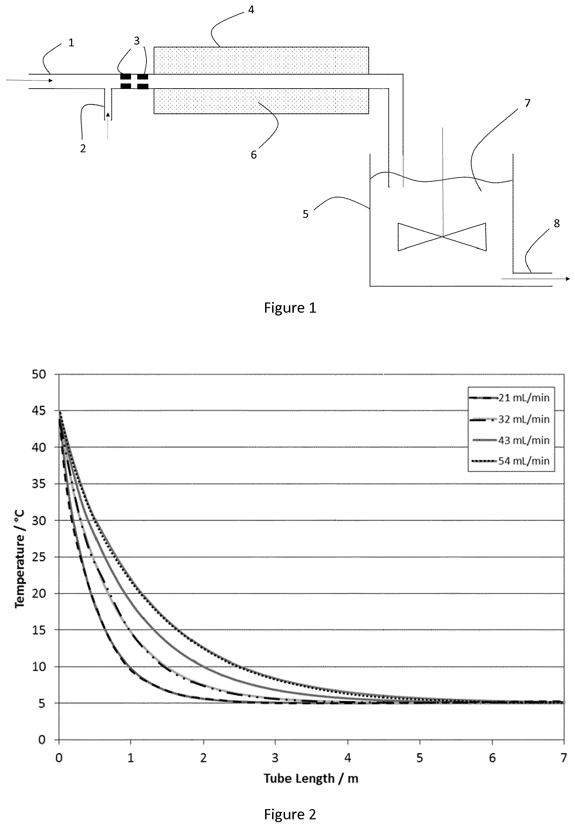

Nucleation can be obtained by either cooling and/or antisolvent injection. Fast cooling rates are achieved by flowing concentrated solutions into these capillaries. The tubular crystallizer is positioned at low temperatures generating highly supersaturated conditions. An example of such device is represented in fig. 1. An antisolvent, or non-solvent is a liquid, at least partially miscible with the solvent which reduces the solubility of the solute.

Depending on the molecule to be crystallised, suitable solvent can be a polar solvent selected from the group consisting of water, alcohol, (methanol, ethanol, propanol, isopropanol, hexanol) acetate (, ethyl acetate, isopropyl acetate,) Ketone (methyl isobutyl ketone, methyl isopropyl ketone) organic acid (acetic acid), acetone, acetonitrile, dimethyl formamide, dimethyl sulfoxide and their mixture or can be apolar solvent such as pentane, hexane, cyclohexane, tetrahydrofuran, benzene, toluene, diethyl ether or others.

Typically, the antisolvent is another liquid selected from the same group consisting of water, alcohol, (methanol, ethanol, propanol, isopropanol, hexanol) acetate (, ethyl acetate, isopropyl acetate,) Ketone (methyl isobutyl ketone, methyl isopropyl ketone) organic acid (acetic acid), acetone, acetonitrile, dimethyl formamide, dimethyl sulfoxide and their mixture or can be apolar solvent such as pentane, hexane, cyclohexane, tetrahydrofuran, benzene, toluene, diethyl ether or others.

Advantageously, cooled antisolvent can be added by a side feed, preferably at constant flow rates. In that case, both the side feed and the main feed are of capillary dimensions, so that fast mixing is obtained, ensuring fast spontaneous nucleation.

Preferably, static mixers, such as restrictions of variable lengths and diameters, included in the flow path prior to cooling, help increasing and controlling the overall nucleation rate; hence, by parameter selection, predefined crystal sizes can easily be obtained with a micrometric dimension (typically between 5µm and 500µm) and a narrow size distribution, both ensuring appropriate biodisponibility and controllable pharmacokinetics.

By restriction, it is meant a change in diameter over a certain distance as opposed to the normal inner diameter of the tubing. Such a restriction can have an inner diameter of 250 to 750 µm, over a length of 1 mm to 50 cm. The purpose of these restrictions is to induce recirculation zones at the end of the restriction in the flow path of the liquid and thus increasing cooling rates further downstream due to convection as well as increasing overall shear rates of the liquid over the full length of the crystallizer. Appropriate positioning of the restrictions renders a full control of crystal nucleation rate and therefore final crystal size.

The dimensions of the capillary ensure fast cooling rates of all the liquid (10-50°C/s) at set flow rate. Length may vary between 15 cm and 10 m: shorter lengths (15 cm to 1m) are used when antisolvent is added to the crystallizing liquid, longer lengths (between 1 and 10 m) when only cooling crystallization is applied.

Supersaturation is the driving force for nucleation. The fast cooling rates and/or antisolvent gradients obtained inside the apparatus provide high supersaturation values resulting in high enough nucleation rates in order to have a continuous stream of small crystals at the outlet of the apparatus. Preferred values between 2 and 18 as supersaturation is defined as σ=(C-C*)/C* with C is the actual concentration and C* the solubility value at the crystallization temperature.

For example, following tests were made on Brivaracetam/IPAc (IsoPropylAcetate) at different concentrations. Induction time (appearance of crystals) is shown in table 1 for different increasing concentrations at a crystallization temperature of 20°C after flowing through the tube at 30 mL/min in a 7m long tubing.

**Table 1**

| **Brivaracetam/IPAc, Solubility at 20°C : 136 mg/mL; solubility at 45°C : ∼1100 mg/mL Flow of 30 mL/min, 7m long tubing, 1 restriction, bath temperature at 20°C, initial temperature at 45°C** | | |
|---|---|---|
| **Concentration mg/mL** | Supersaturation | Appearance of crystals |
| **150** | 0.09 | None |
| **200** | 0.47 | None |
| **250** | 0.83 | 20 h |
| **300** | 1.21 | 2 h |
| **350** | 1.57 | 0.5 h |
| **400** | 1.94 | Immediate |
| **500** | 2.68 | Immediate |
| **600** | 3.41 | Immediate, white slurry coming out of tubing |
| **800** | 4.88 | Immediate, white slurry coming out of tubing |

The combination of flow rates and tube length determines the residence time of the molecules inside the tubing. Moreover, the flow rate impacts the recirculation zones after the restrictions in flow. Therefore, the ideal combination of flow rates and supersaturation time gradient has to be found for each different molecule.

Table 2 shows the change in crystal size for Brivaracetam/IPAc solutions for different flow rates Q at a crystallization temperature Tc of 0°C (so same overall supersaturation). The variation in crystal size is shown without and with a 5 cm long 500 µm restriction. "Needles and rods" indicate appearance of concurring crystallizations of the two possible crystal types of Brivaracetam. Therefore the method also influences selectivity for one single crystal form. In the case of Brivaracetam, rods are preferred.

Decreasing flow rate results in longer residence times and therefore more nucleation, so smaller crystals are observed (tests 2,3 and 4 in table 2). Introducing one single restriction, an optimum is reached for 32 mL/min. Nucleation rates J drastically increased when using one restriction as opposed to none (tests 6,7,8 compared to 2,3,4 in table 2).

**Table 2**

| **Brivaracetam/IPAc, 600 mg/mL, 7 m long tubing, restriction of 5 cm long and inner diameter of 500µm, bath temperature at 0°C, initial temperature at 45°C.** | | | | | | | |
|---|---|---|---|---|---|---|---|
| **N°** | T_{c} | σ | Q | Restriction | Appearance crystals | Crystal length | J |
| | °C | | mL/min | µm | | µm | mL⁻¹.s⁻¹ |
| **1** | 0 | 11 | 54 | none | Needles and rods | | |
| **2** | 0 | 11 | 43 | none | Rods | 1020 ± 196 | 2 100 |
| **3** | 0 | 11 | 32 | none | Rods | 365 ± 106 | 43 000 |
| **4** | 0 | 11 | 21 | none | Rods | 258 ± 76 | 121000 |
| **5** | 0 | 11 | 54 | 500 | Needles and rods | | |
| **6** | 0 | 11 | 43 | 500 | Rods | 227 ± 64 | 181 000 |
| **7** | 0 | 11 | 32 | 500 | Rods | 121 ± 33 | 1 200 000 |
| **8** | 0 | 11 | 21 | 500 | Rods | 175 ± 100 | 400 000 |

The advantage of using restriction(s) has already been indicated in previous tables. A series of tests was also performed using identical conditions and only changing the number of identical restrictions. In table 3, the tests were performed with restrictions of 1 mm in length and having an inner diameter of 500 µm. Crystal length decreases drastically when placing more restrictions in series in the tubing line.

**Table 3**

| Brivaracetam/IPAc, 400 mg/mL, 7 m long tubing, bath temperature at 10°C, initial temperature at 45°C, flow rate of 30 mL/min. used restrictions: 1 mm long and 500 µm inner diameter | | | | | | | | |
|---|---|---|---|---|---|---|---|---|
| N° | T_{c} | σ | Q | Restrictions | Appearance crystals | Crystal length | | J |
| | °C | | mL/min | # | | µm | | mL.⁻¹s⁻¹ |
| 9 | 10 | 4.7 | 30 | 0 | Rods | 195 | ± 62 | 66 000 |
| 10 | 10 | 4.7 | 30 | 1 | Rods | 62 | ± 29 | 3 085 000 |
| 11 | 10 | 4.7 | 30 | 1 | Rods | 62 | ± 20 | 1 609 000 |
| 12 | 10 | 4.7 | 30 | 2 | Rods | 46 | ± 21 | 8 500 000 |
| 13 | 10 | 4.7 | 30 | 2 | Rods | 43 | ± 18 | 7 300 000 |
| 14 | 10 | 4.7 | 30 | 3 | Rods | 37 | ± 19 | 12 000 000 |
| 15 | 10 | 4.7 | 30 | 3 | Rods | 40 | ± 19 | 11 500 000 |
| 16 | 10 | 4.7 | 30 | 4 | Rods | 35 | ± 13 | 9 700 000 |

Also different geometries of restriction were tested on Brivaracetam/IPAc mixtures: different lengths and different inner diameters. All result in fast crystallization and different obtained crystal lengths and sizes, as shown in table 4.

**Table 4**

| Brivaracetam/IPAc, 400 mg/mL, 7 m long tubing, bath temperature at 10°C, initial temperature at 45°C, flow rate of 30 mL/min, one restriction of different lengths and widths | | | | | | | | | |
|---|---|---|---|---|---|---|---|---|---|
| N° | T_{c} | σ | Q | Restriction | | Appearance crystals | Crystal length | | J |
| | | | | length | diameter | | | | |
| | °C | | mL/min | cm | µm | | µm | | mL⁻¹.s⁻¹ |
| 17 | 10 | 4.7 | 30 | 5 | 500 | Rods | 143 | ± 52 | 310 000 |
| 18 | 10 | 4.7 | 30 | 5 | 750 | Rods | 120 | ± 47 | 500 000 |
| 19 | 10 | 4.7 | 30 | 20 | 500 | Rods | 95 | ± 34 | 642 000 |
| 20 | 10 | 4.7 | 30 | 50 | 500 | Rods | 207 | + 45 | 132 000 |
| 21 | 10 | 4.7 | 30 | 100 | 500 | Rods | 211 | ± 53 | 107 000 |
| 9 | 10 | 4.7 | 30 | none | | Rods | 195 | + 62 | 131 000 |

The length of the capillary tube has also been varied. In the following tests, antisolvent hexane at 4°C was added to a solution of Brivaracetam/IPAc. The varying parameters are listed in table 5. It became clear that for these cases different lengths of tubing are to be investigated, as too long tube results into blockage of the tubes due to too high solid content in suspension in the solution, as reported in table 6. In all cases, the preferred rods form was observed.

**Table 5 Anti-solvent test conditions**

| **Parameter** | **Used Values** |
|---|---|
| Concentration (mg.mL⁻¹) | 100, 150, 200, 300 |
| Solution temperature (°C) | 30 |
| Hexane temperature (°C) | 5 |
| Solvent Flow Rate (mL.min⁻¹) | 20 |
| Anti-solvent Flow Rate (mL.min⁻¹) | 20 |
| Tubing inner diameter (µm) | 500 |
| Tubing length (cm) | 18 - 33 - 50 - 100 |
| Temperature crystal growth and filtration (°C) | 25 |

**Table 6**

| N° | Initial concentration Brivaracetam/IPAc mg.mL⁻¹ | σ | Tubular length cm | Crystals Mean maximum length µm |
|---|---|---|---|---|
| 22 | 150 | 8.4 | 18 | 6 ± 2 |
| 23 | 150 | 8.4 | 33 | 9 ± 5 |
| 24 | 150 | 8.4 | 50 | 10 ± 5 |
| 25 | 150 | 8.4 | 50 | 9 ± 4 |
| 26 | 150 | 8.4 | 100 | Blocked tube |
| 27 | 200 | 11.5 | 18 | 12 ± 6 |
| 28 | 200 | 11.5 | 33 | 7 ± 3 |
| 29 | 200 | 11.5 | 50 | 9 ± 5 |
| 30 | 200 | 11.5 | 50 | 7 ± 3 |
| 31 | 200 | 11.5 | 100 | Blocked tube |
| 32 | 300 | 17.8 | 50 | 8 ± 3 |
| 33 | 300 | 17.8 | 100 | Blocked tube |

Tubing length should also be adapted when no antisolvent is added to the molecule/solvent solution. For example in the crystallization of acetylsalicylic acid in Ethanol/water mixtures, it is observed that in tubes longer than 3 m, the solid content in suspension in the solution becomes so high that blockage of the tubing occurs as reported in table 7.

**Table 7**

| Acetylsalicylic acid/ethanol-water, 300 mg/mL, bath temperature at 15°C, initial temperature at 60°C, flow rate of 30 mL/min. used restrictions: 4 restrictions of 1 mm long and 500 µm wide | | | | | | | |
|---|---|---|---|---|---|---|---|
| N° | T_{c} | σ | Q | tubing | | Crystal length | |
| | °C | | mL/min | length m | diameter µm | µm | |
| 34 | 15 | 27 | 30 | 7 | 500 | Tube blocked | |
| 35 | 10 | 27 | 30 | 6 | 750 | Tube blocked | |
| 36 | 10 | 27 | 30 | 5 | 500 | Tube blocked | |
| 37 | 10 | 27 | 30 | 3 | 500 | 800 | ± 68 |
| 38 | 10 | 27 | 30 | 3 | 500 | 820 | ± 80 |
| 39 | 10 | 27 | 30 | 3 | 500 | 795 | ± 73 |

As can be seen from the reported results, depending on the crystallisation system to be treated, different parameters can be varied, and optimal setup depends on the solvent/solute combination and composition.

The crystallization temperature is controlled by a water bath containing about 5L of water, temperature is monitored at all times by a thermocouple. This water is cooled by a 1 m long cupper serpentine plunged into the bath with cooling liquid flowing from and to a cryothermostat, the water bath itself is agitated with a magnetic bar stirrer. The internal volume of a 7m long tubing of 1 mm inner diameter is about 22 mL. As this volume is 220 times smaller than the volume of the present water it is assumed that the water temperature remains within a 0.5°C or smaller margin of the set temperature value.

Especially since the wall thickness of the used tubing is 0.3 mm - PTFE - which enhances temperature exchange between crystallizing liquid and the water bath. Temperature simulations using COMSOL Multiphysics, above mentioned conditions, and - measured and documented - physicochemical values of the solute/solvent solution for heat capacity, heat conductivity and density have pointed out that temperature variation inside the tubing when flowing at different flow rates is fast and that the crystallization temperature of the water bath is reached for flow rates 20 to 50 mL/min, starting from a tube length of 7 m long tubing. Simulated temperature variation as a function of tubing length is shown in figure 2.

### Acetylsalicylic acid Crystallisation

Solvents and solvent mixtures can have an increased viscosity, as is the case for ethanol/water mixtures: viscosity goes through a maximum of when mixed in a 50/50 volumetric ratio. Hence, it is expected that the inner diameter of the restrictions have to be reduced for a mixture of solvent and acetylsalicylic acid to have a positive effect on nucleation rate as opposed to Brivaracetam/Isopropyl acetate solutions.

This reduction in size has to be performed in order to increase the reigning Reynolds number inside the restriction at set flow rates and concentrations. Indeed, in table 8, the average crystal lengths, Sphere Equivalent Diameter (SED) and nucleation rates are shown for 10 repeated tests of several conditions with varying inner diameter and number of restrictions for an acetylsalicylic acid/ethanol/water mixture of 200 mg acetylsalicylic acid/mL solvent in a 50/50 volumetric ratio of ethanol and water.

The use of two restrictions with an inner diameter of 500 µm has some effect on the average size. It is however mostly the use of 250 µm inner diameter restrictions that have a drastic impact: not only is the average crystal length reduced; also the crystal aspect has changed: instead of rectangular rods with an observed equal width and depth for tests 37 to 40, this depth is reduced to about 1/5 of the width in tests 43 and 44.

Therefore, the use of these restrictions has also a large impact on crystal appearance, pinpointing again to the influence of shear stresses on the nucleation behavior of small organic molecules. Next to this, the use of 2 restrictions increases drastically the nucleation rate by 2 orders of magnitude, as was also shown for Brivaracetam. It is expected that any molecule undergoing cooling crystallization in this setup will be influenced by the use and position of proposed flow path restrictions.

**Table 8**

| **Acetylsalicylic acid dissolved in ethanol/water 50/50 vol%, 200 mg/mL, 3 m long tubing, restriction of 500 and 250µm wide and 1 mm long, initial temperature at 60°C, flow rate 30 mL/min, water bath set at 15°C, shown average results from 10 repeated tests where 15 mL of product was collected, filtered and dried.** | | | | | | |
|---|---|---|---|---|---|---|
| **N°** | T_{c} | σ | Restriction type | Crystal length | Sphere Equivalent Diameter | Nucleation rate |
| | °C | | | µm | µm | mL⁻¹.s⁻¹ |
| **40** | 15 | 4.5 | 0 | 1319 ± 167 | 449 ± 74 | 2300 |
| **41** | 15 | 4.5 | 1 of 500 µm ID | 1398 ± 100 | 490 ± 85 | 1900 |
| **42** | 15 | 4.5 | 2 of 500 µm ID | 1049 ± 179 | 348 ± 104 | 3700 |
| **43** | 15 | 4.5 | 1 of 250 µm ID | 962 ± 82 | 328 ± 31 | 5300 |
| **44** | 15 | 4.5 | 2 of 250 µm ID | 393 ± 35 | 117 ± 20 | 130 000 |

### Maturing of crystals.

Using the proposed setup of claim 1, the crystals will have attained a certain size and shape at the end of the tubular crystallizer, which corresponds to their nucleation rate attained inside the tubing. Obtaining different crystal sizes as end-product then depends on the time that is given to the crystals to mature further.

Tests reported in table 9 were performed on acetylsalicylic acid/ethanol/water solutions of 200 mg acetylsalicylic acid/mL solvent in a 50/50 volumetric ratio of ethanol/water, using a flow rate of 30 mL/min and a bath temperature of 10 °C. Crystal growth treatments varied between growth in suspension up to equilibrium followed by filtration and drying (test 45); filtration after 60 seconds (test 46); filtration after 90 seconds (test 47); addition of 250 mL of the mother liquor to 250 mL of a saturated 70 mg/mL acetylsalicylic acid/ethanol/water solution under gentle stirring followed by filtration and drying (test 48).

When the product is filtered after 1 minute, the final product is indeed smaller than the product found at equilibrium. After 90 seconds this effect is already absent, meaning that equilibrium conditions are met very fast inside the liquor. The fact that average crystal length of test 47 is larger than test 45 as to be found in small variations in nucleation rate as a function of time. When the same product is added to a saturated solution, overall concentration drops and therefore the system is more diluted after the nucleation step. Therefore, the number of nuclei formed inside the tubular crystallizer will undergo less growth and hence, reduced crystal sizes are obtained.

**Table 9**

| **Acetylsalicylic acid dissolved in ethanol/water 50/50 vol%, 200 mg/mL, 3 m long tubing, initial temperature at 60°C, flow rate 30 mL/min, water bath set at 10°C, no restrictions used.** | | | | | |
|---|---|---|---|---|---|
| **N°** | T_{c} | σ | Crystal growth treatment | Crystal length | Crystal width |
| | °C | | | µm | µm |
| **45** | 10 | 6.7 | Growth to equilibrium in tubing | 500 ± 230 | 69 ± 27 |
| **46** | 10 | 6.7 | filtration on 16 µm glass filter after 60s | 359 ± 190 | 66 ± 35 |
| **47** | 10 | 6.7 | filtration on 16 µm glass filter after 90s | 646 ± 391 | 38 ± 23 |
| **48** | 10 | 6.7 | Addition to saturated solution | 172 ± 66 | 38 ± 13 |

To further proof the effect of early filtration (see table 10), the liquor of tests 43 and 44 were also directly filtered after exiting the tubing (test 49 and 50, respectively); the product of test 44 was also collected for 2 minutes and filtered afterwards (test 51). As a result, a very fine crystalline powder is obtained which does not require any further processing to be incorporated into tablets.

**Table 10**

| **Acetylsalicylic acid dissolved in ethanol/water 50/50 vol%, 200 mg/mL, 3 m long tubing, restrictions of 250µm wide and 1 mm long, initial temperature at 60°C, flow rate 30 mL/min, water bath set at 15°C. The thickness is set equal to 1/5^{th} of the width (experimentally observed)** | | | | | | | |
|---|---|---|---|---|---|---|---|
| **N°** | T_{c} | σ | Restriction type | Filtration | Crystal length | Crystal width | Sphere Equivalent Diameter |
| | °C | | | | µm | µm | µm |
| **49** | 15 | 4.5 | 1 of 250 µm ID | At exit | 104 ± 55 | 28 ± 13 | 32 ± 7 |
| **50** | 15 | 4.5 | 2 of 250 µm ID | At exit | 63 ± 36 | 23 ± 17 | 24 ± 8 |
| **51** | 15 | 4.5 | 2 of 250 µm ID | After 120s | 155 ± 91 | 36 ± 17 | 42 ± 11 |

## Claims

1. Method for nucleating crystals from a solution comprising the steps of:
- Injecting in a first capillary (1) tube an under saturated solution comprising a solvent and a soluble compound to be crystallised;
- changing the local conditions of the solution downstream of the capillary tube (1) to supersaturated conditions above the metastable conditions, the transition time of the fluid flowing in the capillary tube between the under saturated conditions and the supersaturated conditions above the metastable conditions being less than 1000 ms, preferably below 100 ms, even more preferably less than 10 ms.

2. Method according to claim 1 wherein the transition to supersaturated conditions is obtained by cooling down the capillary wall to temperature below the metastable temperature, and/or injecting a antisolvent in the injected stream, and/or injecting a cooled solvent, the cooled or antisolvent liquid being injected by a second capillary (2) tube having essentially the same dimension as the first capillary.

3. Method according to claim 1 or 2 wherein the first capillary tube (1) has an internal diameter comprised between 1 mm and 100µm, preferably between 800µm and 500µm.

4. Method according to any of the previous claims comprising the step of initially setting the supersaturation between -0.1 (i.e. under saturated) and the high concentration limit of the metastable conditions, supersaturation being defined as the relative difference between the local concentration of the compound to be crystallised and the solubility of the compound to be crystallised.

5. Method according to any of the previous claims wherein the capillary tube comprises static mixing zone(s) (3) improving thermal, antisolvent diffusion and shear rate.

6. Method according to claim 5 wherein the static mixing zone (3) comprises section restrictions, preferably, from one to four restrictions.

7. Method according to claim 6 wherein the inner diameters of the restrictions are comprised between 250 and 750µm, and a length comprised between 1mm and 50cm.

8. Method according to any of claims 5 to 7 wherein the static mixing zone is located upstream of the transition zone between under saturated or metastable conditions and supersaturated conditions above the metastable conditions.

9. Method according to any of the previous claims wherein the supersaturation above the metastable conditions is comprised between 1.6 and 30, preferably between 2 and 18.

10. Method according to any of the previous claims wherein the capillary length is comprised between 15cm and 10m.

11. Method according to any of the previous claims wherein the flow in the capillary length is continuous.

12. Method according to any of the previous claims wherein the flow rate of the under saturated solution is comprised between 2 and 200 mL/min, preferably between 10 and 50mL/min.

13. Method according to any of the previous claims wherein the method is performed as a continuous open loop process.

14. Method according to any of the previous claims wherein the soluble compound is Brivaracetam.

15. Method for crystallising a compound from an under saturated solution comprising the steps of nucleating crystals of the compound by the method according to any of the previous claims and growing the obtained nuclei in a metastable supersaturated solution (7).

16. Method of seeding a supersaturated solution (7) by injecting nuclei obtained by the method of any of the claims 1 to 13.
